(19) Europäisches Patentamt
European Patent Office
Office européen des brevets

(11) Numéro de publication : **0 542 627 A1**

(12) # DEMANDE DE BREVET EUROPEEN

(21) Numéro de dépôt : **92403052.1**

(22) Date de dépôt : **12.11.92**

(51) Int. Cl.$^5$ : **G01N 33/533,** G01N 33/58, G01N 33/543

(30) Priorité : **15.11.91 FR 9114117**

(43) Date de publication de la demande : **19.05.93 Bulletin 93/20**

(84) Etats contractants désignés : **AT BE CH DE DK ES FR GB GR IE IT LI LU MC NL PT SE**

(71) Demandeur : **CLONATEC**
**60, Rue de Wattignies**
**F-75880 Paris Cédex 12 (FR)**

(72) Inventeur : **Samake, Hamidou**
**15 rue des Balkans**
**F-75020 Paris (FR)**
Inventeur : **Goumard, Philippe**
**62 rue Jean Vaquier**
**F-93160 Noisy Le Grand (FR)**
Inventeur : **Sommé, Gérard**
**2 domaine du Vaularon**
**F-91440 Bures Sur Yvette (FR)**

(74) Mandataire : **Orès, Bernard et al**
**Cabinet ORES 6, Avenue de Messine**
**F-75008 Paris (FR)**

(54) **Réactif de diagnostic, ses applications dans un procédé pour la détermination d'un analyte et dispositif pour la mise en oeuvre dudit procédé.**

(57) Réactif de diagnostic et ses applications dans un procédé pour la détermination qualitative et quantitative rapide d'un ligand ou analyte dans un fluide à tester ainsi qu'un dispositif pour la mise en oeuvre dudit procédé.

Ledit réactif répond à la formule **I** ci-après :

$$L\text{-}r\text{-}s\text{-}Col\text{-}(S)_n \qquad (I)$$

dans laquelle **L** représente une substance de liaison apte à se lier de manière spécifique soit à un ligand à détecter dans un fluide, soit à un récepteur dudit ligand ; **r** est un groupe réactif assurant la liaison entre le colorant Col et la substance L, lequel groupe r est apte à réagir par addition, substitution ou tout autre réaction avec la substance L ; **s** constitue le support du groupe réactif et est du type $(XH)_x$-$(Y)_y$-$(Z)_z$, dans laquelle X représente un atome d'azote, Y représente un groupe CO ou un groupe $SO_2$, Z représente un groupe $CH_2$, x et y représentent 0 ou 1, et z représente 0, 1 ou 2 ; **Col** est une molécule apte à produire une coloration ;

**S** représente un ou plusieurs groupes solubilisants de type $SO_3X'$, COOX' ou $NX'^+$, dans lesquels X' représente un atome d'hydrogène ou un atome de métal alcalin ; **n** représente 0 ou 1.

EP 0 542 627 A1

Jouve, 18, rue Saint-Denis, 75001 PARIS

La présente invention est relative à un réactif de diagnostic et à ses applications dans un procédé pour la détermination qualitative et quantitative rapide d'un ligand ou analyte dans un fluide à tester.

La présente invention est également relative à un dispositif pour la mise en oeuvre dudit procédé.

L'invention s'applique plus particulièrement, mais non limitativement, à la détection de la présence d'anticorps ou d'antigènes dans un fluide biologique, à la détection de substances toxiques, de virus ou de bactéries dans un fluide quelconque.

Il existe un besoin, toujours actuel, de disposer aussi bien pour les dosages immunologiques que pour la détection d'acides nucléiques, de conjugués marqués plus performants que ceux connus à ce jour (conjugués enzymatiques ou radiomarqués), permettant une diminution du nombre d'étapes dans les procédés mis en oeuvre, notamment à fin d'automatisation, une diminution du bruit de fond et présentant une plus grande stabilité que les conjugués enzymatiques actuellement disponibles, tout en présentant au moins une sensibilité égale sinon supérieure auxdits conjugués actuels.

Un nombre important de Brevets ou de Demandes de Brevets fournissent une vaste littérature des différentes techniques de production de conjugués ou signaux discernables dans les immunoessais.

La Demande de Brevet européen 205 326 IMMUNOMEDICS INC., décrit des anticorps modifiés par un polymère synthétique de type poly(amide/urée/thiourée) (PAUT) homogène contenant notamment des atomes de bore, de telle sorte que le conjugué obtenu soit utile en radiothérapie.

L'anticorps décrit dans cette Demande présente une structure du type Ac-(lr-PAUT)$_n$, dans laquelle Ac est l'anticorps, lr est un élément de liaison entre l'anticorps et le poly(amide/urée/thiourée), tel qu'un ester, un amide, de l'urée, un carbamate ou une thiourée.

La Demande de Brevet européen 347 139 EASTMAN KODAK Corp. décrit une composition à base également de leucoimidazole, comprenant de l'hydroxy-4' acétanilide, à une concentration jusqu'à 2,5 mmolaire.

Une telle composition peut être utilisée dans un test immunologique dans lequel est également mis en oeuvre un conjugué marqué à la peroxydase.

Le complexe marqué formé dans l'essai est alors détecté par addition de la composition formant un colorant, qui va se colorer en présence de la peroxydase et de son substrat tel que $H_2O_2$.

L'utilisation d'une telle composition a l'avantage de permettre d'augmenter la concentration en conjugué marqué à l'enzyme, de détecter de faibles concentrations d'antigènes et de diminuer la durée de l'essai, sans pour autant modifier la sensibilité ni augmenter le bruit de fond.

Cependant, tous les conjugués marqués de l'Art antérieur ont l'inconvénient majeur de nécessiter une étape de révélation et d'entraîner ainsi :

. soit des manipulations accrues, qui sont des sources d'erreur dans les tests "domestiques" ;

. soit des difficultés dans l'automatisation des tests de dosage.

La présente invention s'est en conséquence donné pour but de pourvoir à un réactif coloré prêt à l'emploi, stable et permettant d'éviter l'étape de révélation avec un chromogène, la lecture du résultat étant en effet directe.

Un tel réactif est notamment utilisable dans toutes les réactions du type ligand-récepteur, dans lesquelles le ligand est défini comme toute substance dont la détection peut présenter un intérêt et le récepteur est défini comme toute substance capable de se lier de manière spécifique à un ligand.

On peut citer, à titre d'exemple, comme récepteur toute molécule de nature polymérique, protéique, polypeptidique, glucidique, lipidique, dont les propriétés physicochimiques ou biologiques présentent un intérêt dans le domaine du diagnostic, de l'immunologie, de la biologie moléculaire, de l'anatomopathologie, de l'immunohistochimie ou de la biochimie.

La présente invention a pour objet un réactif coloré, caractérisé en ce qu'il répond à la formule I ci-après :

$$L\text{-}r\text{-}s\text{-}Col\text{-}(S)_n \qquad (I)$$

dans laquelle

L représente une substance de liaison apte à se lier de manière spécifique soit à un ligand à détecter dans un fluide, soit à un récepteur dudit ligand ;

r est un groupe réactif assurant la liaison entre le colorant Col et la substance L, lequel groupe r est apte à réagir par addition, substitution ou tout autre réaction avec la substance L ;

s constitue le support du groupe réactif et est du type $(XH)_x$-$(Y)_y$-$(Z)_z$, dans laquelle
X représente un atome d'azote,
Y représente un groupe CO ou un groupe $SO_2$,
Z représente un groupe $CH_2$,
x et y représentent 0 ou 1, et
z représente 0, 1 ou 2 ;

Col est une molécule chimique dont la structure lui confère la propriété d'absorber une lumière à une ou plusieurs longueurs d'ondes situées dans l'UV, le visible ou l'IR ;

S représente un ou plusieurs groupes solubilisants de type $SO_3X'$, COOX' ou $NX'^{+}$, dans lesquels X' représente un atome d'hydrogène ou un atome de métal alcalin ;

n représente 0 ou 1.

De manière préférée :

. L est un anticorps, un antigène, un acide nucléique ou toute autre molécule apte à se lier au ligand recherché, ou à un récepteur de ce dernier, de formule du type $R'-NH_2$ ou R'-OH, R' constituant le résidu d'un acide aminé, d'un peptide, d'une protéine, d'un acide nucléique ou de toute autre molécule appropriée.

. r est un groupe réactif susceptible de réagir avec les substances L (anticorps, antigènes, acide nucléique, etc...) et est notamment choisi parmi :

* les groupes aliphatiques éventuellement substitués ; parmi les substituants possibles, on peut citer les atomes d'halogènes conduisant notamment à des mono et polyhalogénures d'alkyle, le groupe COOR", conduisant notamment à des esters et plus particulièrement à des esters sulfuriques ou sulfoniques,
* des groupes aromatiques tels que les halogénonitrobenzènes, les benzoylazides, les sulfofluorures, ou
* des groupes hétérocycliques tels que des dérivés de chlorure de cyanuryle (chlorotriazine, notamment), tétrachloropyrimidine, trichloropyrimidine, trifluorochloropyrimidine, dichloroquinoxaline, dichloroquinazoline, dichlorophtalazine, dichloropyridazine, chlorobenzothiazole,

. Col est avantageusement choisi parmi les dérivés azoïques, les dérivés anthraquinoniques, les phtalocyanines, les dérivés sulfonés ou des groupes organométalliques possédant un substituant métal carbonyl. Par exemple, si l'on souhaite l'obtention d'une coloration jaune, on choisira plutôt les azoïques et les phénylazopyrazoloniques ; pour l'obtention d'une coloration orangée ou rouge, les monoazoïques dérivés de l'acide J et de l'acide H ; pour l'obtention d'une coloration bleue, les dérivés de l'amino-1 anilino-4 anthraquinone ; pour l'obtention d'une coloration bleue, turquoise ou verte, les phtalocyanines de cuivre et de nickel ; et pour l'obtention d'une coloration violette, bleue, brune, grise et noire, les complexes de cuivre, de chrome et de cobalt de la série azoïque.

On peut citer, à titre d'exemple, un certain nombre de colorants commercialisés :
Procion®, Procion H®, Procilan® (ICI) ; Cibacron®, Lanasol® (CIBA) ; Remazol® (HOECHST) ; Levafix®, Verofix® (BAYER) ; Reacton®, Reactofil® (GEIGY) ; Drimaren®, Drimalan F® (SANOZ) ; Primazin® (BASF) ; Solidazol® (CASSELLA) ; Reatex® (FRANCOLOR).

De manière surprenante, de tels réactifs substance de liaison-colorant, lors de leur mise en oeuvre dans un procédé de diagnostic, ont l'avantage d'augmenter la sensibilité et la réactivité dudit procédé de manière significative par rapport à un procédé dans lequel le colorant est fixé sur des particules (EP 165 633) ainsi que de permettre d'effectuer ladite détection en une seule étape ; ils peuvent également être en outre avantageusement couplés à une moitié de paire d'affinité.

De tels réactifs conformes à l'invention trouvent notamment application dans :

a) des tests de diagnostic rapides qui font intervenir, comme support solide, des membranes de forte capacité de liaison (tests rapides de type filtration, par exemple).

Un réactif anticorps-colorant, par exemple, peut être utilisé pour révéler une réaction immunologique antigène-anticorps, dans le cadre :

- soit d'une méthode sandwich, dans les détections d'antigènes bactériens ou viraux, dans les détections d'hormones polyspécifiques, de marqueurs tumoraux ou de différenciation cellulaire, de cytokines ;
- soit d'une méthode de type capture, notamment en sérologie ;
- ou soit d'une méthode de type compétition, notamment pour les détections d'hormones et de médicaments.

Le réactif conforme à l'invention permet, dans tous les cas, la lecture directe du résultat sur le support utilisé.

b) des tests rapides, de type migration :

Dans le cas d'un réactif conforme à l'invention de type anticorps-colorant, ce dernier est arrêté, après migration éventuelle, par une molécule bloquante déposée sur le support solide. Les applications de ces tests sont les mêmes que celles qui sont décrites pour les tests rapides de type filtration.

Les réactifs conformes à l'invention trouvent également application :

. dans les tests de type ELISA (compétition directe ou indirecte, sandwich, immunocapture) qui font intervenir des supports de type billes de polystyrène, tubes, plaques de microtitration, ailettes, etc..., et dans lesquels le réactif conforme à l'invention, par exemple un réactif anticorps-colorant est utilisé à la place du conjugué enzymatique, fluorescent ou (chemi)luminescent;

. dans des tests d'agglutination dans lesquels les particules sont sensibilisées avec un réactif conforme à l'invention ;

. dans des tests du type Western blot ou RIBA, dans lesquels des anticorps spécifiquement liés à des protéines d'un antigène recherché sont visualisés avec un réactif conforme à l'invention ;

. dans des tests en phase homogène dans lesquels plusieurs leucodérivés, associés aux substances à détecter entraînent le développement d'une coloration.

La présente invention a également pour objet un procédé de préparation du réactif coloré conforme à l'invention, caractérisé en ce qu'il comprend le couplage d'un colorant de formule r-s-Col-$(S)_n$, dans laquelle r, s, Col, S et n ont la même signification que ci-dessus avec une substance de liaison L,
lequel couplage est réalisé par réaction entre r et L.

Les groupements réactifs r peuvent réagir directement avec la substance de liaison L ou indirectement, après modification chimique (activation) ou par l'intermédiaire de réactifs bifonctionnels.

Selon un mode de mise en oeuvre avantageux dudit procédé, ledit couplage est réalisé par réaction directe par un processus de substitution par une β- ou une γ-élimination.

Conformément à l'invention, le couplage par réaction directe par substitution, peut être réalisé en mettant en oeuvre l'une quelconque des réactions suivantes :

a. le couplage d'un colorant portant un groupe dichloro-2-3 quinoxaline de formule A :

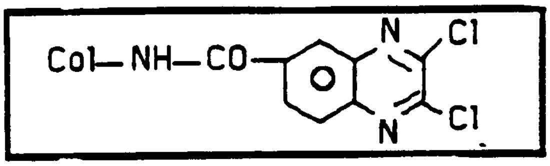

formule A

avec une substance de liaison de type R'-$NH_2$ ou R'-OH, conformément au schéma 1 ci -après :

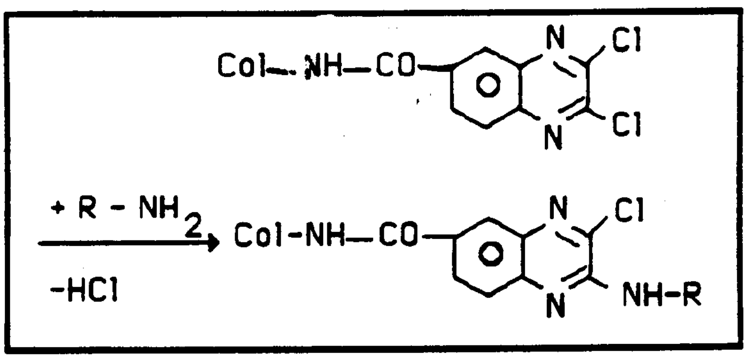


schéma 1

b. le couplage d'un colorant chlorotriazinique de formule B :

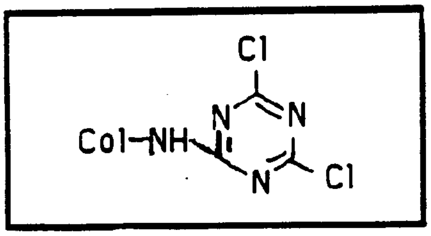

formule B

avec une substance de liaison de type R'-$NH_2$ ou R'-OH, conformément au schéma 2 ci -après :

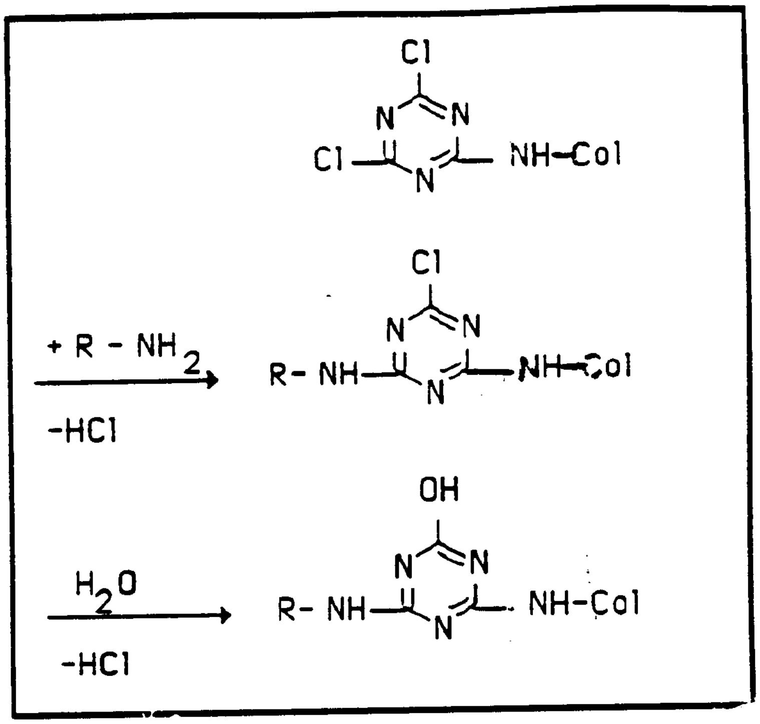


schéma 2

c. le couplage d'un colorant tétrachloro-2-4-5-6 pyrimidine de formule C :

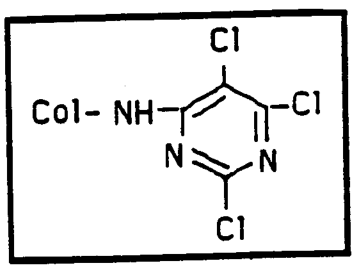

formule C

avec une substance de liaison récepteur de type R'-$NH_2$ ou R'-OH, conformément au schéma 3 ci-après :

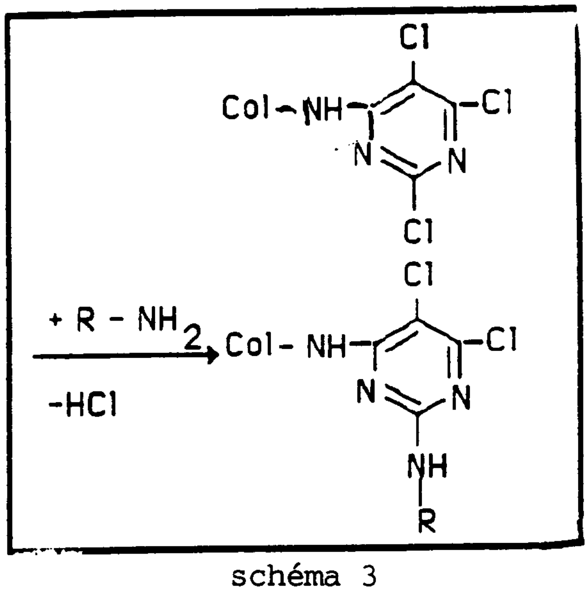


schéma 3

d. le couplage d'un colorant dérivé du chloro-2 benzothiozole de formule D :

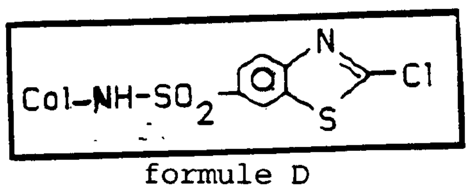

formule D

avec une substance de liaison de type $R\text{-}NH_2$ ou R-OH, conformément au schéma 4 ci-après :

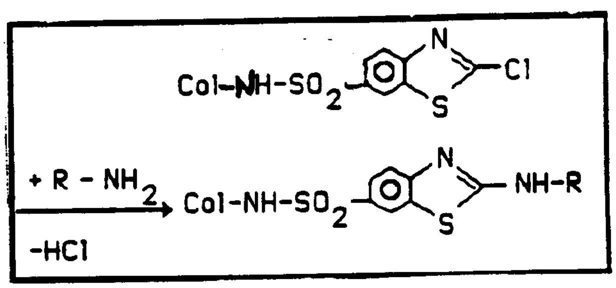


schéma 4

D'autres colorants tels que

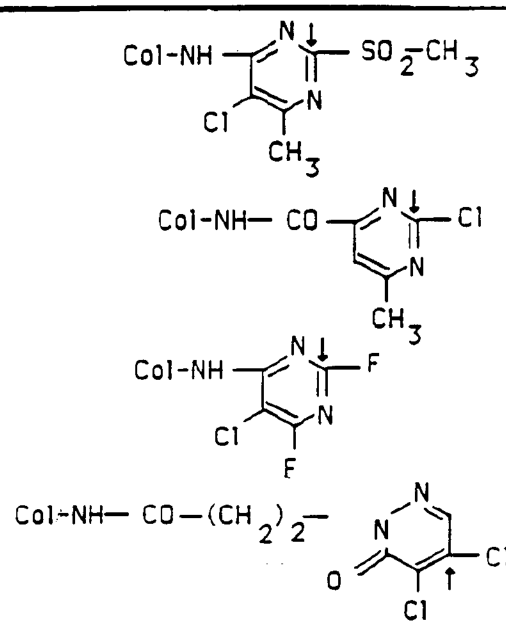

Levafix P (Bayer)

Reactofil (Geigy)

Verofix (Bayer)
Drimalan (Sandoz)

Primazin P (BASF)
(pour laine et polyamide)

(La flèche indique la position où s'effectue la substitution)

peuvent également être couplés à une substance de liaison de type $R\text{-}NH_2$ ou R-OH par une réaction directe par substitution.

Selon un autre mode de mise en oeuvre avantageux dudit procédé, ledit couplage par réaction directe comprend une réaction d'élimination suivie d'une réaction d'addition.

Conformément à l'invention, le couplage par réaction directe par élimination suivie d'addition peut être réalisé comme suit :

a. le couplage d'un colorant comprenant un groupe β-sulfatoéthyle sulfonamide ou un groupe N-(β- ou γ-chloroalkylsulfonamide) de formule E :

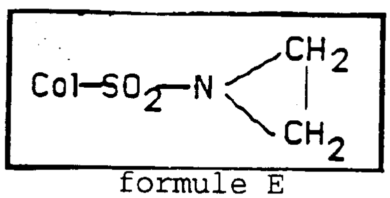

formule E

avec une substance de liaison de type $R'\text{-}NH_2$ ou R'-OH, conformément au schéma 5 ci -après :

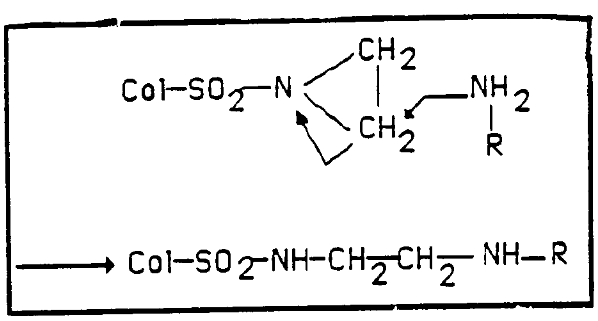

schéma 5

b. le couplage d'un colorant comprenant un reste β-sulfatoéthylsulfone de formule F :

$$Col-SO_2-CH_2-CH_2-O-SO_3H$$

formule F

avec une substance de liaison de type R'-$NH_2$ ou R'-OH, conformément au schéma 6, ci-après :

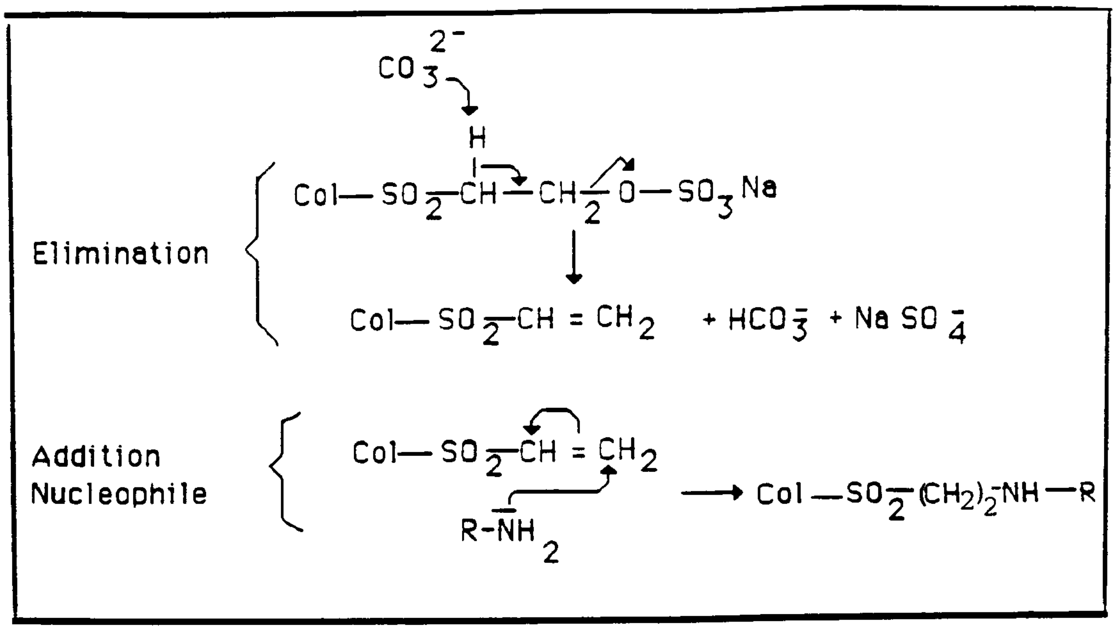


schéma 6

Selon encore un autre mode de mise en oeuvre avantageux dudit procédé, ledit couplage est réalisé par réaction indirecte, en effectuant l'activation du colorant et/ou de la substance de liaison, préalablement à la réaction entre r et L.

On peut citer, à titre d'exemples non limitatifs :

- pour l'activation du colorant, l'action du N-hydroxysuccinimide sur un colorant porteur d'un groupement COOH ou l'action de réactifs bifonctionnels tels que le m-maléimidobenzoyl-N-hydroxysulfosuccinimide ester ou l'iminothiolane-HCl,
- pour l'activation de la substance de liaison, une oxydation ménagée des glycanes d'une glycoprotéine, une activation par un groupement bifonctionnel tels que le sulfosuccinimidyl-4(N-maléimidométhyl)cyclohexane-1 carboxylate, le N-hydroxysuccinimidyl-4-ozidobenzoate ou le N-succinimidyl 3(2-pyridyldithio)propionate.

La présente invention a également pour objet un procédé de détection qualitative et quantitative rapide de la présence d'un ligand dans un fluide, du type comprenant la mise en contact d'un échantillon contenant éventuellement le ligand à détecter avec au moins une substance de liaison spécifique, dont au moins l'une d'entre elles est un récepteur du ligand, caractérisé en ce qu'une desdites substances de liaison spécifique (qui se lie soit au ligand, soit à un récepteur du ligand) est couplée à un colorant de formule r-s-Col-$(S)_n$ pour former un réactif conforme à l'invention.

Selon un mode de mise en oeuvre avantageux dudit procédé, au moins l'une des substances de liaison spécifique est fixée sur un support solide approprié.

On entend par support solide, au sens de la présente invention, aussi bien des billes (polyacrylamide, verre, polystyrène,...) que des particules, des tubes, des disques, des membranes, des bandelettes ou des microplaques.

Selon un autre mode de mise en oeuvre avantageux dudit procédé, l'ensemble des réactifs est fixé sur un support solide approprié ; un tel support est dit réactif.

Selon une disposition avantageuse de ce mode de mise en oeuvre, au moins l'un des réactifs est apte à migrer sur ledit support.

Selon une autre disposition avantageuse de ce mode de mise en oeuvre, au moins l'un des réactifs est en position fixe sur ledit support.

De manière avantageuse, ledit support est une membrane réactive à faible rétention des protéines.

Conformément à ce dernier mode de mise en oeuvre, le procédé comprend au moins :

(1) la mise en contact de l'échantillon à analyser avec une substance de liaison spécifique du ligand à détecter (récepteur) couplée à une moitié de paire d'affinité et/ou à un colorant de formule r-s-Col-$(S)_n$, apte à migrer sur ledit support,

(2) la mise en contact du complexe formé en (1) avec une substance fixée sur ledit support, choisie dans le groupe qui comprend l'autre moitié de la paire d'affinité, une substance de liaison spécifique du ligand et une substance de liaison spécifique du récepteur, et

(3) la lecture directe de la réaction.

De manière avantageuse, dans le cas d'une méthode directe, lorsque le test est positif, le ligand présent dans l'échantillon réagit avec le réactif récepteur couplé à une moitié de paire d'affinité et/ou un colorant de formule r-s-Col-$(S)_n$ et permet notamment d'obtenir un effet de concentration du ligand ; puis le complexe ligand/récepteur marqué réagit avec le réactif de l'étape (2), fixé sur le support, qui arrête la migration dudit ligand et permet la lecture directe du résultat (présence d'une coloration au niveau de la fenêtre de lecture).

Par contre, lorsque le test est négatif, le complexe n'est pas formé et ne migre donc pas ; en ce cas, il n'y a aucune coloration au niveau de la fenêtre de lecture.

On peut citer notamment comme paires d'affinité, les paires biotine-avidine ou streptavidine, dérivé de métal lourd-groupe thio, divers homopolynucléotides comme poly dG-poly dC, poly dA-poly dT et poly dA-poly dU, haptène-anticorps anti-haptène, enzyme-substrat, enzyme-inhibiteur, lectine-glycane.

Le nombre de réactifs mis en oeuvre dans le procédé conforme à l'invention dépend essentiellement de la méthode mise en oeuvre (sandwich, directe, compétition, capture notamment).

En variante, ledit procédé comprend :

(1) la mise en contact de l'échantillon à analyser avec une substance de liaison spécifique du ligand à détecter (récepteur) couplée à une moitié de paire d'affinité,

(2) la mise en contact de l'ensemble ligand/récepteur-moitié de paire d'affinité avec un réactif conforme à l'invention L-r-s-Col-$(S)_n$ puis

(3) la mise en contact du complexe formé en (2) avec l'autre moitié de la paire d'affinité et

(4) la lecture directe de la réaction.

La présente invention a également pour objet un kit de diagnostic prêt à l'emploi pour la mise en oeuvre du procédé de détection d'un ligand conforme à l'invention, caractérisé en ce qu'il comprend au moins :

- des doses appropriées d'un réactif substance de liaison spécifique-colorant conforme à l'invention éventuellement couplé à une moitié de paire d'affinité ; et
- des quantités utiles de tampons et autres réactifs convenables pour la mise en oeuvre de ladite détection.

La présente invention a, en outre, pour objet un dispositif pour la détermination qualitative et quantitative rapide de la présence d'un ligand, dans un fluide, du type comprenant une zone de réaction contenant un réactif destiné à réagir avec le ligand recherché et une zone de révélation, lequel dispositif est caractérisé en ce qu'il comprend :

- une zone de réaction (a) qui comprend une membrane activée prévue pour recevoir un échantillon de fluide à tester et destinée à être associée à au moins une substance de liaison spécifique du ligand à détecter (récepteur) convenablement couplée à une moitié de paire d'affinité, et/ou à un colorant de formule r-s-Col-$(S)_n$, apte à migrer ;
- une zone de révélation associée à une substance fixée sur ledit support, choisie dans le groupe qui comprend l'autre moitié de la paire d'affinité, une substance de liaison spécifique du ligand et une substance de liaison spécifique du récepteur et dans laquelle le complexe éventuellement formé dans la zone (a) est fixé.

Selon un mode de réalisation avantageux dudit dispositif, entre la zone de réaction (a) et la zone de révélation, une zone de contrôle du bon fonctionnement du test est prévue et comprend une phase solide convenable contenant un réactif de référence.

Selon une disposition avantageuse de ce mode de réalisation, le réactif de référence, non marqué, est apte à reconnaître un réactif de la zone de réaction (a) et peut notamment être identique au ligand que l'on cherche à détecter.

Conformément à l'invention la substance de liaison couplée à une moitié d'une paire d'affinité et/ou à un colorant de formule I est un récepteur du ligand recherché, notamment un antigène, un anticorps ou un acide nucléique.

En variante, ledit dispositif comprend :

- une première zone de réaction qui comprend une membrane activée prévue pour recevoir un échantillon de fluide à tester et destinée à être associée à au moins un réactif convenablement couplé à une moitié de paire d'affinité, apte à reconnaître un ligand recherché et à migrer ;
- une deuxième zone de réaction qui comprend une membrane activée qui est associée à un réactif coloré conforme à l'invention, apte à se lier au complexe formé dans la première zone de réaction, et
- une zone de révélation associée à l'autre moitié de paire d'affinité et dans laquelle le complexe éventuellement formé dans la première zone et coloré dans la deuxième zone est fixé.

Selon un mode de réalisation avantageux dudit dispositif, entre la première et la deuxième zone de réaction, une zone de contrôle du bon fonctionnement du test est prévue et comprend- une phase solide convenable contenant un réactif de référence.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

**EXEMPLE 1 : Couplage d'un anticorps polyclonal (Ac) et d'un colorant activé de type β-sulfatoéthylsulfone (Royal Blue EFR® BAYER).**

- Protocole :

On dialyse la quantité nécessaire d'Ac, contre du tampon carbonate-bicarbonate 0,1 M pH 9,5 pendant 3 heures.

On prépare une solution à 5 mg de colorant par ml de carbonate bicarbonate 0,1 M pH 9,5.

On fait un rapport de marquage de 2 mg d'Ac pour 0,75 mg de colorant, de manière à obtenir une concentration finale à 2 mg/ml en anticorps. Le couplage dure toute la nuit sous agitation à température ambiante.

Puis, le conjugué obtenu est purifié, par chromatographie de gel filtration sur colonne de GF05 IBF équilibrée préalablement dans du tampon carbonate bicarbonate 0,1 M pH 9,5 (2 mg de gel/1 mg conjugué).

La caractérisation du conjugué se fait dans un premier temps par chromatographie HPLC sur une colonne TSK sur SW3000 équilibrée en PBS.

La mesure des densités optiques à 280 nm et 600 nm, permet de calculer les concentrations en anticorps et en colorant. La courbe de la densité optique en fonction de la concentration en colorant obéit à la loi de béer-Lambert et permet de déterminer le coefficient d'extinction molaire du colorant ($\varepsilon$ = 0,12 à 600 nm).

La concentration en colorant (C) est donné par la relation suivante :

$$CC\ (mg/ml) = DO_{600} \times 0,12$$

La concentration en anticorps est donnée par la relation suivante :

$$C_{AC}\ (mg/ml) = \frac{DO_{280} - (DO_{600} \times 0,12)}{1,4}$$

Le conjugué ainsi obtenu est stocké à +4°C dans l'attente de son utilisation.

**EXEMPLE 2 : Couplage comprenant une activation du colorant (couplage indirect).**

Action du N-hydroxysuccinimide sur un colorant portant une fonction carboxylique.

- Activation :

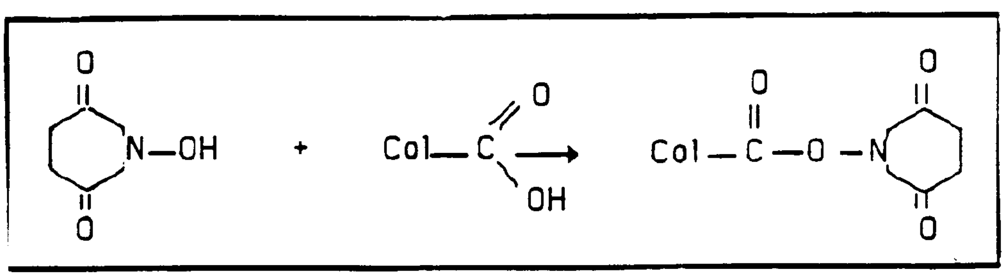

- Mécanisme de la réaction :

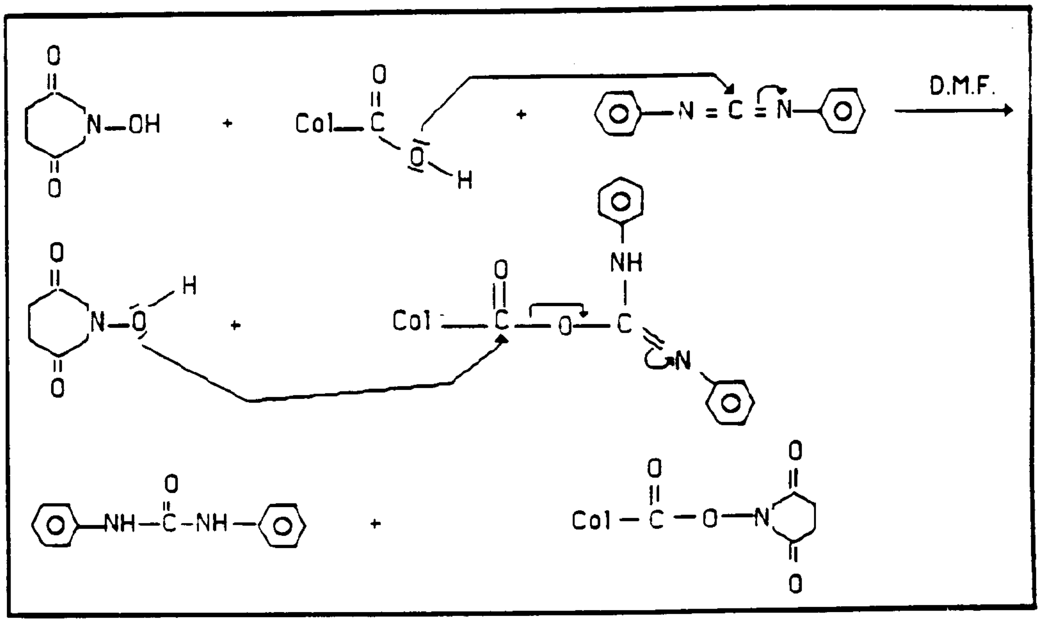


- Couplage proprement dit :

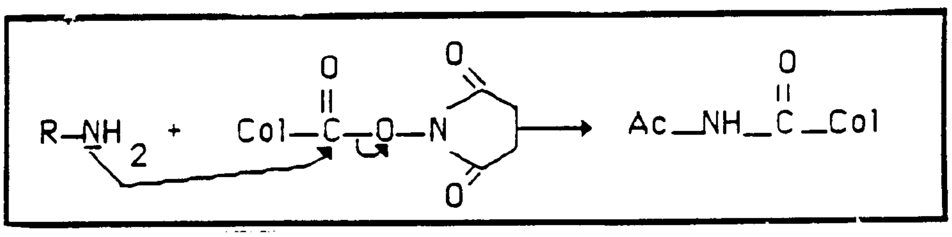

**EXEMPLE 3 : Couplage comprenant une activation de la substance de liaison spécifique (couplage indirect).**

Activation par oxydation ménagée des glycanes d'une glycoprotéine.

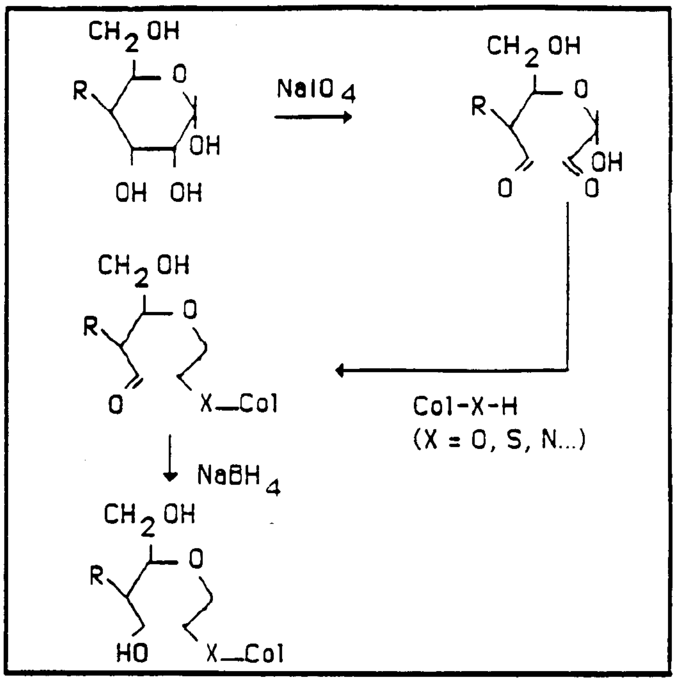


1. Activation de la glycoprotéine (anticorps) :

On dialyse 5 mg d'anticorps contre 1 000 volumes d'une solution de bicarbonate de sodium 100 mM, 2 heures à température ambiante.

On ajoute une solution de $NaIO_4$ 100 mM en quantité suffisante pour obtenir une molarité de 10 mM et on incube 2 heures à température ambiante.

2. Couplage du colorant à la protéine :

On ajoute 5 mg de crésyl violet en PBS pH 7,2. On ajuste le pH du mélange réactionnel à 9,5 avec du tampon carbonate 1 M pH 9,5. On vérifie le pH.

On incube 3 heures à 18°C. On arrête la réaction avec 1/20ème de volume de solution de $NaBH_4$ 132 mM préparée extemporanément, puis on laisse en contact 30 minutes à température ambiante.

On ajoute lentement et sous agitation 1/2 volume de solution de sulfate d'ammonium saturée. On laisse en contact 15 heures à +4°C. On centrifuge à 10 000 g pendant 10 minutes à +4°C, puis on reprend le précipité dans 0,2 ml de tampon Tris 0,1 M, NaCl 1 M, pH 8,0.

**EXEMPLE 4 : Couplage du Crésyl violet perchlorate® KODAK avec un anticorps.**

1. Préparation et activation du colorant :

Elle se fait selon le principe de l'exemple 2, dans les conditions suivantes : on ajoute 10 mg de colorant pour 100 µl de DMF + 400 µl de tampon phosphate 0,1 M pH 7,0 à 10 mg S.SMCC pour 0,15 ml tampon phosphate 0,1 M pH 7 ; on incube 60 minutes à +30°C, avec agitation du tube toutes les 5 minutes ; on filtre sur Millex® 0,8 µm ; on purifie sur une colonne HR 10/10 Fast flow PHARMACIA conditionnée en tampon phosphate 0,1 M pH 6, 5 mM EDTA, dont la boucle d'injection est de 1 ml et le débit de 120 ml/h ; on élue avec le tampon de conditionnement et l'on collecte le premier pic, qui peut être concentré sur cône AMICON CF 25® 2 500 tr/min.

On obtient un volume final ≦ 1,3 ml.

Le colorant est mis en attente en attendant le couplage à 4°C dans l'obscurité.

2. Activation de l'anticorps :

- 10 mg d'anticorps conditionnés en PBS pH 7,4 50 mM phosphate 150 mM NaCl sont concentrés à 8-12 mg/ml. On ajoute 16 µg de SPDP (5-4-N-Succinimidyl 3-(2-pyridyldithio)propionate) par mg d'anticorps, à 30 mM dans l'éthanol absolu.
  On incube 30 min sur agitateur à rouleaux.
  On filtre sur Millex® 0,8 µm. On purifie sur colonne HR 10/10 Fast flow® PHARMACIA conditionnée en PBS pH 7,4 50 mM phosphate 150 mM NaCl, dont la boucle d'injection est 1 ml et le débit de 120 ml/h, puis on élue dans le tampon de conditionnement et l'on collecte le premier pic.
- Concentration :
  Sur cône AMICON CF 25® à 2 500 tr/min, le volume final doit être ≧ 1,3 ml.
- Réduction des anticorps au dithiotréitol (DTT).
- Préparation :
  Un volume de DTT 0,5 M (77,1 mg/ml) en PBS pH 7,4 50 mM phosphate, 150 mM NaCl égal à 1/20ème du volume d'anticorps est ajouté à la solution Ig-SPDP obtenue ci-dessus. On incube 18-20 min à température ambiante sous agitation, puis on filtre sur Millex® 0,8 µm.
- Purification :
  Elle est effectuée sur colonne HR 10/10 conditionnée en phosphate 0,1 M pH 6, 5 mM EDTA et dont la boucle d'injection est de 1 ml et le débit de 120 ml/h. L'élution est réalisée en phosphate : 0,1 M pH 6, 5 mM EDTA et on collecte le premier pic.
- Qualification :
  Mesurer l'absorbance à 280 nm.

$$\text{Concentration : [IgG] mg/ml} = \frac{DO_{280}}{1,4}$$

3. Couplage :

Le couplage de 0,15 mg de colorant par mg d'anticorps est effectué une nuit à +4°C à l'obscurité sur un agitateur à rouleaux à vitesse lente.

La réaction est arrêtée avec 25 % en volume de N-éthylamine 0,12 M (12,51 mg/ml) dans un tampon phosphate 0,1 M pH 6, 5 mM EDTA, pendant 30 minutes à température ambiante sur rouleaux.

Le conjugué est purifié par chromatographie sur GF05 en tampon carbonate bicarbonate 0,1 M pH 9,5.

La caractérisation en colorant est donnée par la relation suivante : CC (mg/ml) = $DO_{600}$ x 0,12.

La concentration en anticorps est donnée par la relation suivante :

$$\text{CAc (mg/ml)} = \frac{DO_{280} - (DO_{260} x 0,12)}{1,4}$$

Les étapes du couplage sont résumées dans le schéma suivant :

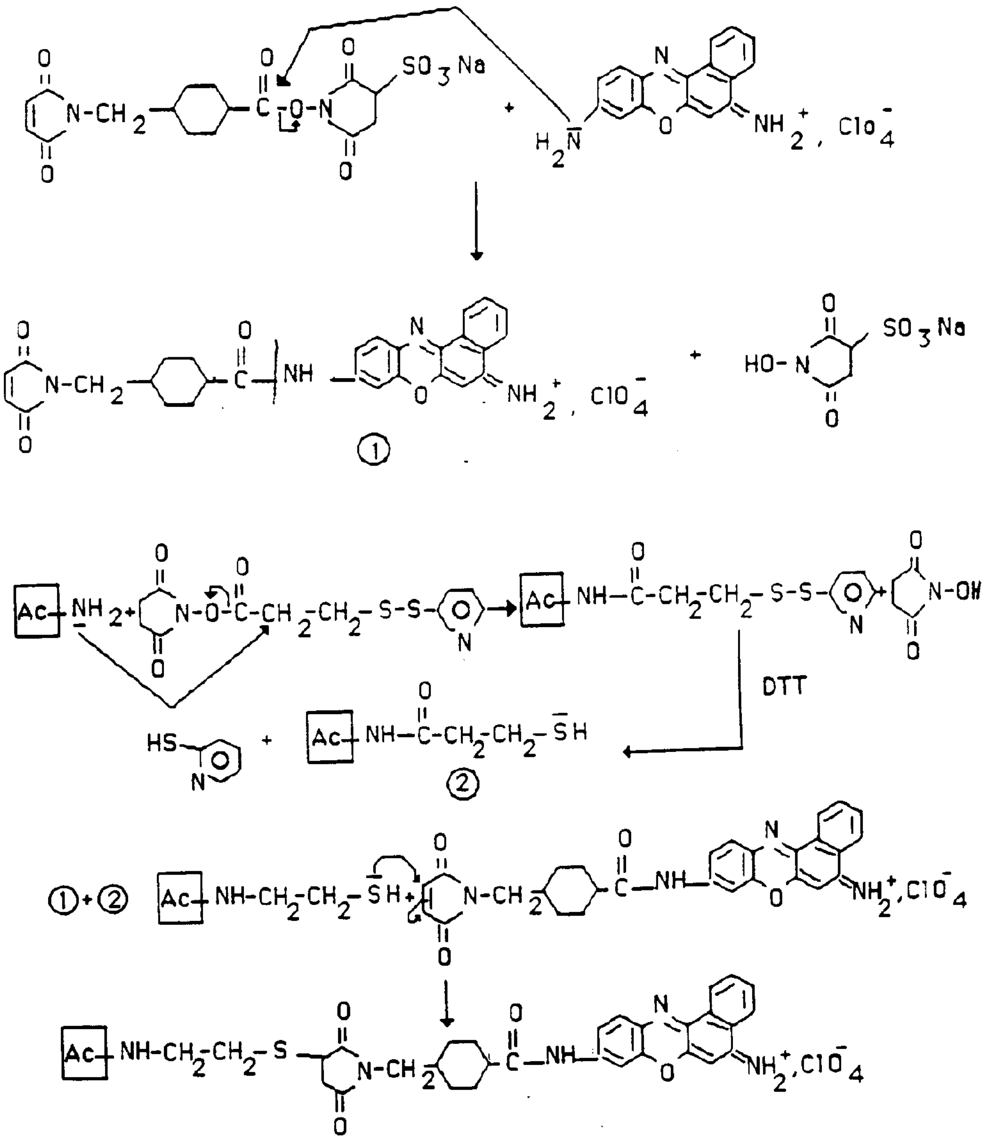


**EXEMPLE 5 : Test rapide par immunofiltration.**

- Principe du test :

  Il s'agit d'un test immunoenzymatique rapide pour la détection et la discrimination des anticorps anti-HIV1 et anti-HIV2 dans le plasma, le sérum ou le sang total du type CLONATEC RAPID HIV1/HIV2 Ab. L'immunofiltre se compose d'un cylindre de plastique sur lequel est fixée une membrane réactive. Tous les réactifs filtrés à travers la membrane sont piégés à l'intérieur du cylindre de l'immunofiltre. Le principe repose sur la capture des anticorps spécifiques par deux peptides de synthèse homologues, dérivés des glycoprotéines transmembranaires des virus HIV1 et HIV2 (peptides identiques à ceux décrits dans le kit CLONATEC RAPID HIV1/HIV2 Ab®). Ces deux peptides sont fixés sur la membrane filtrante en deux points diamétralement opposés (spots HIV1 et HIV2). L'échantillon biologique dilué est filtré à travers la membrane réactive. Les anticorps spécifiques retenus par les peptides synthétiques sont détectés par addition d'un anticorps polyclonal anti-IgG humaines couplé au Bleu Royal EFR.
- Composition de la trousse :

Les réactifs fournis dans la trousse permettent de réaliser 30 tests :

R1 : immunofiltres prêts à l'emploi,
R2 : diluant des échantillons lyophilisé,
R3 : tampon de reconstitution de R2,
R4 : traceur coloré (conjugué anti-IgG-Bleu Royal EFR),
R5 : tampon de reconstitution de R4,
R6 à R8 : sérums de contrôle (négatif, positif faible et positif fort).

- Mode Opératoire :

Après avoir procédé à la reconstitution des réactifs lyophilisés et avoir ramené tous les constituants de la trousse à température ambiante, les échantillons à tester sont dilués au 1/10 dans le diluant prévu à cet effet.

1. Déposer 300 µl (6 gouttes) de l'échantillon dilué sur la membrane et laisser filtrer.
2. Déposer 150 µl (3 gouttes) du traceur coloré. Après filtration, attendre deux minutes.
3. Déposer 1 ml d'eau distillée. Filtrer.

- Interprétation des résultats :

1. Validité du test :

Dans tous les cas d'interprétation, un anneau bleu périphérique, témoin du bon déroulement des opérations et du bon fonctionnement du test doit apparaître sur la membrane. En cas d'absence de ce témoin, le test doit être refait.

2. Interprétation du test :

Un résultat est négatif si aucun spot bleu n'est visible sur la membrane.

Un résultat est positif si au moins un spot bleu apparaît sur la membrane. Selon sa position, il indique le type d'anticorps détecté :

- anti-HIV1 en position 1
- anti-HIV2 en position 2

Le pourcentage de réactivité croisée entre les protéines d'enveloppe des deux virus n'étant pas nul, il est possible de voir apparaître les deux spots pour quelques sérums isolés : le diagnostic de l'infection par HIV1 ou HIV2 sera donné par le spot le plus intense.

- Résultats :

Le Tableau I rassemble les résultats obtenus avec quelques sérums testés à l'aide de la trousse décrite ci-dessus et selon le mode opératoire décrit.

Sept sérums anti-HIV1 dont 4 séroconversions, deux sérums anti-HIV2 et cinq sérums anti-HIV négatifs ont été testés en respectant le mode opératoire de la trousse enzymatique, l'étape de révélation par un chromogène étant dans ce cas supprimée.

TABLEAU I

| Sérum anti-HIV1 | Spot HIV1 | Spot HIV2 | BF |
|---|---|---|---|
| 1 | +/- | - | - |
| 2 | + | - | - |
| 3 | + | - | - |
| 4 | ++ | - | - |
| 5 | ++ | - | - |
| 6 | ++ | - | - |
| 7 | ++ | - | - |

| Sérum anti-HIV2 | Spot HIV1 | Spot HIV2 | BF |
|---|---|---|---|
| 8 | - | +++ | +/- |
| 9 | - | + | - |

| Sérum anti-HIV- | Spot HIV1 | Spot HIV2 | BF |
|---|---|---|---|
| 10 | - | - | - |
| 11 | - | - | - |
| 12 | - | - | - |
| 13 | - | - | - |
| 14 | - | - | - |

Sérums n° 1 à 4 : séroconversions anti-HIV1
Sérums n° 5 à 7 : sérums anti-HIV1
Sérums n° 8 à 9 : sérums anti-HIV2
Sérums n° 10 à 14 : donneurs de sang négatifs en anticorps HIV.
BF : bruit de fond sur la membrane
Echelle de lecture : - : négatif ; +/- : positif faible ; +/++ : positifs croissants.

**EXEMPLE 6 : Détection des anticorps anti-HIV à l'aide d'un test comportant un réactif conforme à l'invention. Comparaison avec un test comportant des particules de latex.**

- Principe du test :
  Il s'agit d'un test rapide par immunochromatographie avec réactifs intégrés pour la détection anti-HIV.

Le test se compose d'une bandelette sur laquelle est fixée une membrane activée. Le principe repose sur la capture des anticorps spécifiques par un peptide de synthèse fixé sur la membrane. L'échantillon biologique est mis en contact avec la membrane réactive. Les anticorps spécifiques se fixent sur le peptide synthétique et sont détectés par des molécules conjuguées à un colorant (réactif conforme à l'invention) conformément à la figure 1, ou à des particules de latex (réactif témoin). Si l'échantillon contient des anticorps anti-HIV une bande colorée apparaît en fin de réaction.

- Protocole :

20 membranes sensibilisées sont mises en contact avec 10 sérums contenant des anticorps anti-HIV1 et 10 sérums négatifs en anticorps anti-HIV. Le traceur est soit un conjugué coloré conforme à l'invention (Tableau II), soit des particules de latex (Tableau III).

- Résultats :

Tableau II : une bande colorée d'intensité "+" apparaît avec les 10 sérums HIV positifs testés, sans bruit de fond significatif sur la membrane. Aucun signal, ni aucun bruit de fond sur la membrane ne sont observés avec les 10 sérums HIV négatifs testés.

Tableau III : Seuls 4 sérums sur les 10 sérums HIV positifs testés sont révélés avec ce système dont 3 faiblement (+/-). Parmi les 10 sérums anti-HIV négatifs testés, 4 échantillons apparaissent positifs dans le système avec une intensité (+/-).

- Intérêt du traceur coloré conforme à l'invention par rapport aux particules de latex :

Avec les 20 sérums testés, les résultats montrent que l'utilisation d'un traceur coloré permet à la fois d'obtenir une meilleure spécificité et une sensibilité satisfaisante, comparativement à l'emploi des particules de latex. De plus, l'utilisation des particules de latex présente en outre l'inconvénient d'une technique de sensibilisation des particules de latex plus longue, comparativement à celle des molécules colorées.

Par ailleurs, l'utilisation du réactif conforme à l'invention permet l'obtention d'un résultat plus rapide et a l'avantage d'être d'un coût réduit.

TABLEAU II

| Sérum HIV + | Signal | Bruit de fond |
|---|---|---|
| 1356,91 | + | - |
| 1357,91 | + | - |
| 1360,91 | + | - |
| 1361,91 | + | - |
| 1362,91 | + | - |
| 1363,91 | + | - |
| 1364,91 | + | - |
| 1365,91 | + | - |
| 1367,91 | + | - |
| 1368,91 | + | - |

| Sérum HIV - | Signal | Bruit de fond |
|---|---|---|
| 326100 | - | - |
| 7329279 | - | - |
| 8325207 | - | - |
| 332518 | - | - |
| 6325211 | - | - |
| 8326101 | - | - |
| 326081 | - | - |
| 2326085 | - | - |
| 3325221 | - | - |
| 2329309 | - | - |

Echelle de lecture : - = négatif
+- = faible
+ = positif
++ = positif fort

TABLEAU III

| Sérum HIV + | Signal | Bruit de fond |
|---|---|---|
| 1356,91 | - | - |
| 1357,91 | - | - |
| 1360,91 | - | - |
| 1361,91 | - | - |
| 1362,91 | +- | - |
| 1363,91 | + | - |
| 1364,91 | +- | - |
| 1365,91 | +- | - |
| 1367,91 | - | - |
| 1368,91 | - | - |

| Sérum HIV - | Signal | Bruit de fond |
|---|---|---|
| 326100 | - | - |
| 7329279 | - | - |
| 8325207 | - | - |
| 332518 | +- | - |
| 6325211 | - | - |
| 8326101 | - | - |
| 326081 | - | - |
| 2326085 | +- | - |
| 3325221 | +- | - |
| 2329309 | +- | - |

Echelle de lecture : - = négatif
+- = faible
+ = positif
++ = positif fort

**<u>EXEMPLE 7</u> : dosage d'HCG avec le procédé conforme à l'invention.**

On prépare un réactif conforme à l'invention selon les protocoles précisés ci-dessus :

- cas d'une méthode de type sandwich :

le réactif conforme à l'invention est un anticorps anti-HCG conjugué à un colorant tel que défini ci-dessus; la migration a lieu, conformément à la figure 2a, sur une membrane de nitrocellulose fixée sur une feuille de Mylar®.

Le dépôt du sérum à tester s'effectue en (1) ; en présence d'HCG dans ledit sérum testé ; un complexe HCG anticorps 1 anti-HCG-colorant (complexe 1) se forme en (2) et migre jusqu'au niveau de l'anticorps 2 anti-HCG fixé en (3), où il est arrêté par la formation d'un complexe 2 entre l'HCG du complexe 1 et l'anticorps 2 anti-HCG fixé ; une partie de l'anticorps 1 anti-HCG-colorant se fixe au niveau du témoin de fonctionnement (4), qui est de l'HCG ; le résultat positif est matérialisé par un signe "+" coloré.

En l'absence d'HCG, l'anticorps 1 anti-HCG-colorant (2) migre et se fixe au niveau du témoin de fonctionnement (4) (HCG fixé) ; ce résultat négatif est matérialisé par un signe "|" coloré.

- cas de l'utilisation d'une paire d'affinité streptavidine-biotine :

  le réactif conforme à l'invention est un anticorps anti-HCG-colorant-biotine ; la migration a lieu conformément à la figure 2b, sur le même type de membrane que ci-dessus.

  Le dépôt du sérum à tester s'effectue en (1) ; en présence d'HCG dans ledit sérum testé, un complexe HCG anti-HCG-colorant-biotine (complexe 1) se forme en (2) et migre ; ce complexe 1 saturé en HCG, ne se fixe pas au niveau de l'HCG fixé (3) et migre jusqu'au niveau de la streptavidine (4) où il est arrêté par la formation d'un complexe biotine-streptavidine ; en (2), en raison de la présence à la fois d'un anti-HCG-colorant-biotine et d'anticorps de souris-colorant, les anticorps de souris-colorant se fixent au niveau du témoin de fonctionnement (5) correspondant à des anticorps anti-souris ; le résultat positif est alors matérialisé par un signe "+" coloré.

  En l'absence d'HCG, les anticorps de souris-colorant présents en (2) migrent et se fixent au témoin de fonctionnement anti-souris en (5) ; ce résultat négatif est matérialisé par un signe "|" coloré.

  Le colorant est représenté par le signe "X" sur les figures 2a et 2b.

**EXEMPLE 8 : dosage des hormones thyroïdiennes.**

On prépare un réactif conforme à l'invention selon les protocoles précisés ci-dessus :

- cas de l'utilisation d'une paire d'affinité streptavidine-biotine par une méthode directe :

  le réactif conforme à l'invention est un anticorps anti-hormones thyroïdiennes (T3 ou T4) conjugué à un colorant tel que défini ci-dessus ; la migration a lieu, conformément à la figure 3a, sur une membrane de nitrocellulose fixée sur une feuille de Mylar®.

  Le dépôt du sérum à tester s'effectue en (1) ; en présence d'hormones thyroïdiennes (HT), un complexe HT anti HT-colorant-biotine se forme en (2) et migre jusqu'en (4) où se forme un complexe biotine-streptavidine; le résultat positif est matérialisé par un signe "-" coloré.

  En l'absence d'HT, le réactif conforme à l'invention anti HT-colorant-biotine présent en (2) migre et forme un complexe avec l'HT fixé en (3) ; le résultat négatif est matérialisé par l'absence de signe en (4).

  On peut prévoir un témoin de bon fonctionnement comme dans l'exemple 7.

- cas de l'utilisation d'une paire d'affinité streptavidine-biotine dans une méthode de compétition :

  le réactif conforme à l'invention est un anticorps anti HT-colorant-biotine ; la migration a lieu conformément à la figure 3b, sur le même type de membrane que ci-dessus.

  Le dépôt du sérum à tester s'effectue en (1) ; en présence d'HT dans ledit sérum, cette dernière se fixe en (3) au conjugué anti HT-colorant et est en compétition avec le conjugué HT-biotine déposé en (2) ; un résultat est positif en l'absence de coloration car le conjugué HT-biotine, qui migre en (4) et entraîne la formation d'un complexe biotine-streptavidine n'est pas coloré.

  En l'absence d'HT (ou HT en défaut), le résultat négatif est matérialisé par la présence d'une coloration en (4) car le conjugué HT-biotine (2) en migrant a formé un complexe avec l'anti HT-colorant (3) avant de s'arrêter en (4).

  Le colorant est représenté par le signe "X" sur les figures 3a et 3b.

  Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

**1°)** Réactif coloré, caractérisé en ce qu'il répond à la formule I ci-après :

$$L\text{-}r\text{-}s\text{-}Col\text{-}(S)_n \qquad (I)$$

dans laquelle

L représente une substance de liaison apte à se lier de manière spécifique soit à un ligand à détecter dans un fluide, soit à un récepteur dudit ligand ;

r est un groupe réactif assurant la liaison entre le colorant Col et la substance L, lequel groupe r est apte à réagir par addition, substitution ou tout autre réaction avec la substance L ;

s constitue le support du groupe réactif et est du type $(XH)_x$-$(Y)_y$-$(Z)_z$, dans laquelle
X représente un atome d'azote,
Y représente un groupe CO ou un groupe $SO_2$,
Z représente un groupe $CH_2$,
x et y représentent 0 ou 1, et
z représente 0, 1 ou 2 ;

Col est une molécule chimique dont la structure lui confère la propriété d'absorber une lumière à une ou plusieurs longueurs d'ondes situées dans l'UV, le visible ou l'IR ;

S représente un ou plusieurs groupes solubilisants de type $SO_3X'$, COOX'ou $NX'^{+}$, dans lesquels X' représente un atome d'hydrogène ou un atome de métal alcalin ;

n représente 0 ou 1.

**2°)** Réactif selon la revendication 1, caractérisé en ce que :

. L est un anticorps, un antigène, un acide nucléique ou toute autre molécule apte à se lier au ligand recherché, ou à un récepteur de ce dernier, de formule du type $R'$-$NH_2$ ou R'-OH, R' constituant le résidu d'un acide aminé, d'un peptide, d'une protéine, d'un acide nucléique ou de toute autre molécule appropriée.

. r est un groupe réactif susceptible de réagir avec les substances L (anticorps, antigènes, acide nucléique, etc...) et est notamment choisi parmi :

* les groupes aliphatiques éventuellement substitués ; parmi les substituants possibles, on peut citer les atomes d'halogènes conduisant notamment à des mono et polyhalogénures d'alkyle, le groupe COOR", conduisant notamment à des esters et plus particulièrement à des esters sulfuriques ou sulfoniques,
* des groupes aromatiques tels que les halogénonitrobenzènes, les benzoylazides, les sulfofluorures, ou
* des groupes hétérocycliques tels que des dérivés de chlorure de cyanuryle, tétrachloropyrimidine, trichloropyrimidine, trifluorochloropyrimidine, dichloroquinoxaline, dichloroquinazoline, dichlorophtalazine, dichloropyridazine, chlorobenzothiazole,

. Col est avantageusement choisi parmi les dérivés azoïques, les dérivés anthraquinoniques, les phtalocyanines, les dérivés sulfonés ou des groupes organométalliques possédant un substituant métal carbonyl.

**3°)** Réactif selon la revendication 1 ou la revendication 2, caractérisé en ce qu'il est en outre couplé à une moitié de paire d'affinité.

**4°)** Procédé de préparation du réactif coloré selon l'une quelconque des revendications 1 à 3, caractérisé en ce qu'il comprend le couplage d'un colorant de formule r-s-Col-$(S)_n$, dans laquelle r, s, Col, S et n ont la même signification que ci-dessus avec une substance de liaison L, lequel couplage est réalisé par réaction entre r et L.

**5°)** Procédé selon la revendication 4, caractérisé en ce que ledit couplage est réalisé par réaction directe par un processus de substitution par une β- ou une γ-élimination.

**6°)** Procédé selon la revendication 5, caractérisé en ce que le couplage par réaction directe par substitution, peut être mis en oeuvre par l'une quelconque des réactions suivantes :

a. le couplage d'un colorant portant un groupe dichloro-2-3 quinoxaline de formule A :

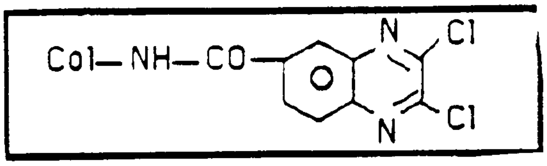

formule A

avec une substance de liaison de type R'-$NH_2$ ou R'-OH, conformément au schéma 1 ci -après :

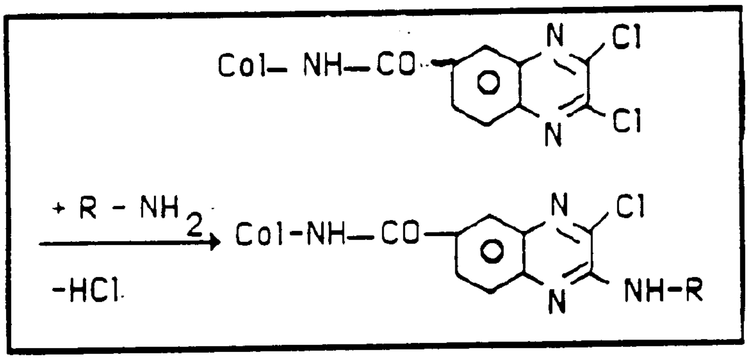


schéma 1

b. le couplage d'un colorant chlorotriazinique de formule B :

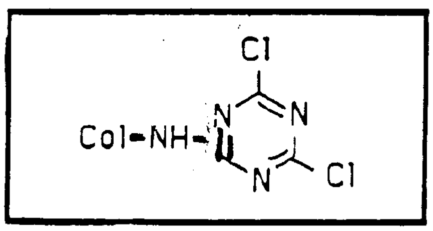

formule B

avec une substance de liaison de type R'-$NH_2$ ou R'-OH, conformément au schéma 2 ci-après :

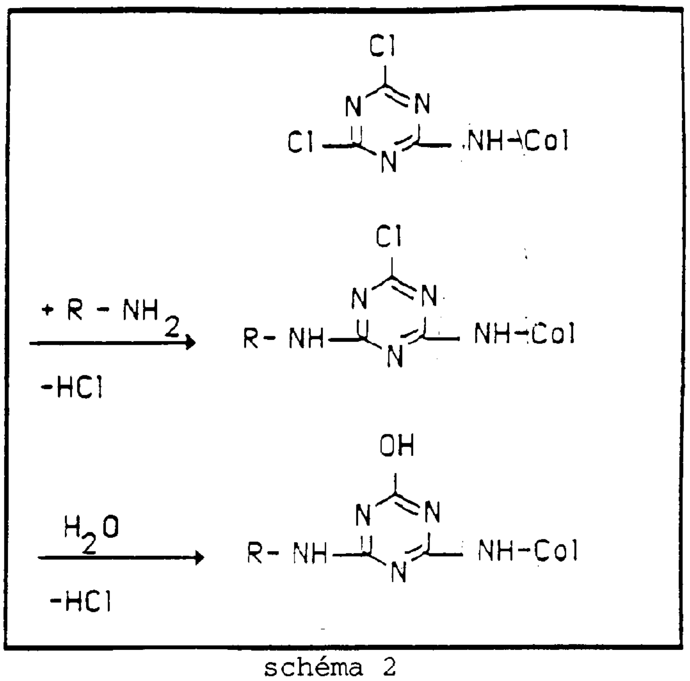


schéma 2

c. le couplage d'un colorant tétrachloro-2-4-5-6 pyrimidine de formule C :

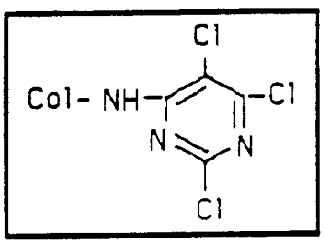

formule C

avec une substance de liaison récepteur de type R'-$NH_2$ ou R'-OH, conformément au schéma 3 ci-après :

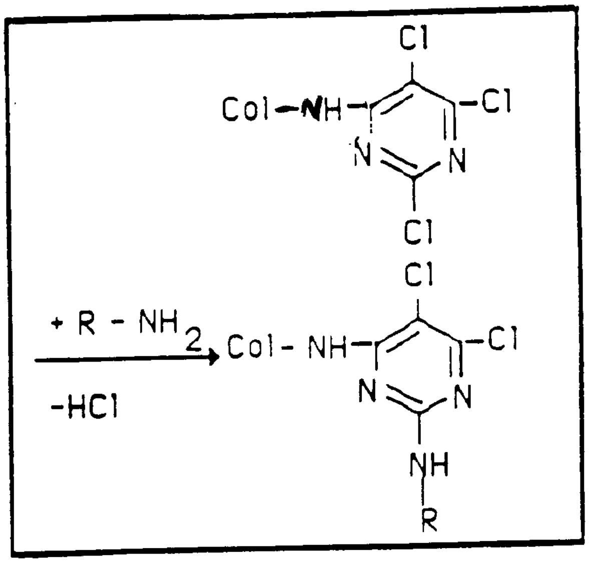


schéma 3

d. le couplage d'un colorant dérivé du chloro-2 benzothiozole de formule D :

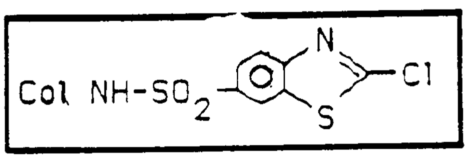

formule D

avec une substance de liaison de type R-$NH_2$ ou R-OH, conformément au schéma 4 ci-après :

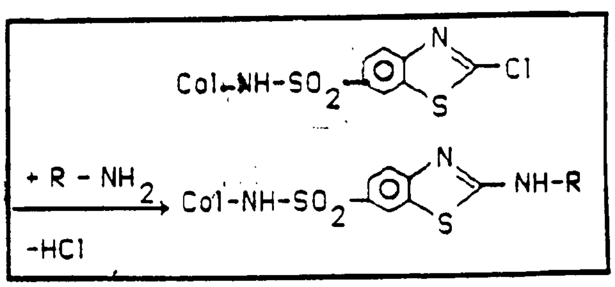


schéma 4

**7°)** Procédé selon la revendication 4, caractérisé en ce que ledit couplage par réaction directe comprend une réaction d'élimination suivie d'une réaction d'addition.

**8°)** Procédé selon la revendication 7, caractérisé en ce que le couplage par réaction directe par élimination suivie d'addition peut être mis en oeuvre par l'une quelconque des réactions suivantes :

a. le couplage d'un colorant comprenant un groupe β-sulfatoéthyle sulfonamide ou un groupe N- (β- ou γ-chloroalkylsulfonamide) de formule E :

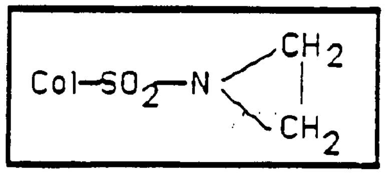

formule E

avec une substance de liaison de type R'-$NH_2$ ou R'-OH, conformément au schéma 5 ci -après :

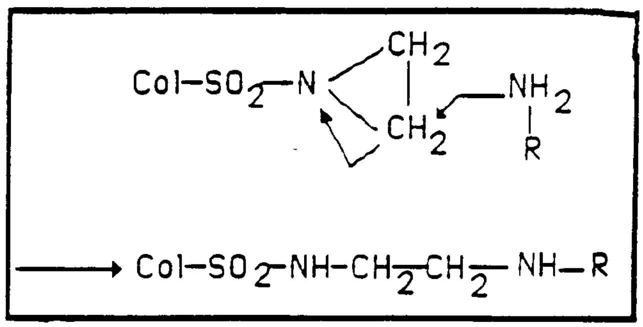

schéma 5

b. le couplage d'un colorant comprenant un reste β-sulfatoéthylsulfone de formule F :

$$Col-SO_2-CH_2-CH_2-O-SO_3H$$

formule F

avec une substance de liaison de type R'-$NH_2$ ou R'-OH, conformément au schéma 6, ci-après :

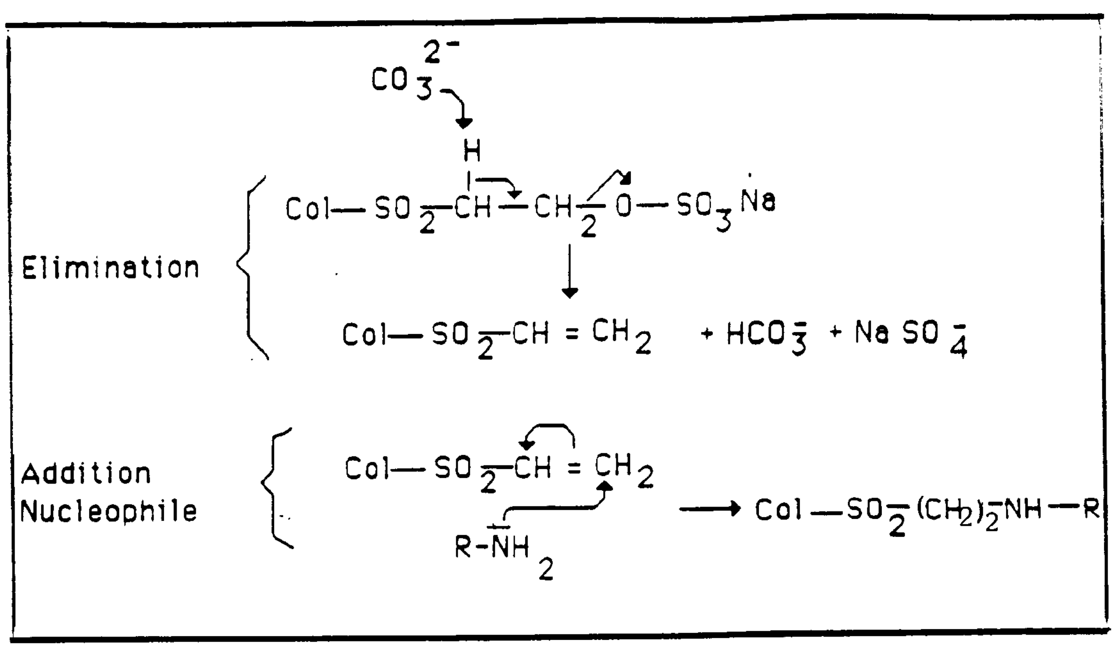


schéma 6

**9°)** Procédé selon l'une quelconque des revendications 4 à 8, caractérisé en ce que ledit couplage est réalisé par réaction indirecte en effectuant l'activation du colorant et/ou de la substance de liaison, préalablement à la réaction entre r et L.

**10°)** Procédé de détection qualitative et quantitative rapide de la présence d'un ligand dans un fluide, du type comprenant la mise en contact d'un échantillon contenant éventuellement le ligand à détecter avec au moins une substance de liaison spécifique, dont au moins l'une d'entre elles est un récepteur du ligand, caractérisé en ce qu'une desdites substances de liaison spécifique (qui se lie soit au ligand, soit à un récepteur du ligand) est couplée à un colorant de formule r-s-Col-$(S)_n$ pour former un réactif selon l'une quelconque des revendications 1 à 3.

**11°)** Procédé selon la revendication 10, caractérisé en ce qu'au moins l'une des substances de liaison spécifique est fixée sur un support solide approprié.

**12°)** Procédé selon la revendication 10, caractérisé en ce que l'ensemble des réactifs est fixé sur un support solide approprié.

**13°)** Procédé selon la revendication 12, caractérisé en ce qu'au moins l'un des réactifs est apte à migrer sur ledit support.

**14°)** Procédé selon la revendication 12, caractérisé en ce qu'au moins l'un des réactifs est en position fixe sur ledit support.

**15°)** Procédé selon l'une quelconque des revendications 12 à 14, caractérisé en ce que le support est une membrane à faible rétention des protéines.

**16°)** Procédé selon la revendication 12, caractérisé en ce qu'il comprend au moins :

(1) la mise en contact de l'échantillon à analyser avec une substance de liaison spécifique du ligand à détecter (récepteur) couplée à une moitié de paire d'affinité et/ou à un colorant de formule r-s-Col-$(S)_n$, apte à migrer sur ledit support,

(2) la mise en contact du complexe formé en (1) avec une substance fixée sur ledit support, choisie dans le groupe qui comprend l'autre moitié de la paire d'affinité, une substance de liaison spécifique du ligand et une substance de liaison spécifique du récepteur, et

(3) la lecture directe de la réaction.

**17°)** Procédé de détection qualitative et quantitative rapide d'un ligand, caractérisé en ce qu'il comprend :

(1) la mise en contact de l'échantillon à analyser avec une substance de liaison spécifique du ligand à détecter (récepteur) couplée à une moitié de paire d'affinité,

(2) la mise en contact de l'ensemble ligand/récepteur-moitié de paire d'affinité avec un réactif selon l'une quelconque des revendications 1 à 3, puis

(3) la mise en contact du complexe formé en (2) avec l'autre moitié de la paire d'affinité et

(4) la lecture directe de la réaction.

**18°)** Kit de diagnostic prêt à l'emploi pour la mise en oeuvre du procédé de détection d'un ligand selon l'une quelconque des revendications 10 à 17, caractérisé en ce qu'il comprend au moins :

- des doses appropriées d'un réactif selon l'une quelconques des revendications 1 à 3 ; et
- des quantités utiles de tampons et autres réactifs convenables pour la mise en oeuvre de ladite détection.

**19°)** Dispositif pour la détermination qualitative et quantitative rapide de la présence d'un ligand, dans un fluide, du type comprenant une zone de réaction contenant un réactif destiné à réagir avec le ligand recherché et une zone de révélation, lequel dispositif est caractérisé en ce qu'il comprend :

- une zone de réaction (a) qui comprend une membrane activée prévue pour recevoir un échantillon de fluide à tester et destinée à être associée à au moins une substance de liaison spécifique du ligand à détecter (récepteur) convenablement couplée à une moitié de paire d'affinité, et/ou à un colorant de formule r-s-Col-$(S)_n$, apte à migrer ;
- une zone de révélation associée à une substance fixée sur ledit support, choisie dans le groupe qui comprend l'autre moitié de la paire d'affinité, une substance de liaison spécifique du ligand et une substance de liaison spécifique du récepteur et dans laquelle le complexe éventuellement formé dans la zone (a) est fixé.

**20°)** Dispositif selon la revendication 19, caractérisé en ce que entre la zone de réaction (a) et la zone de révélation, une zone de contrôle du bon fonctionnement du test est prévue et comprend une phase solide convenable contenant un réactif de référence.

**21°)** Dispositif selon la revendication 19, caractérisé en ce que le réactif de référence, non marqué est apte à reconnaître un réactif de la zone de réaction (a) et peut notamment être identique au ligand que l'on cherche à détecter.

**22°)** Dispositif selon l'une quelconque des revendications 19 à 21, caractérisé en ce que la substance de liaison couplée à une moitié d'une paire d'affinité et/ou à un colorant de formule I est un récepteur du ligand

recherché, notamment un antigène, un anticorps ou un acide nucléique.

**23°)** Dispositif pour la détermination qualitative et quantitative rapide de la présence d'un ligand, caractérisé en ce qu'il comprend :

- une première zone de réaction qui comprend une membrane activée prévue-pour recevoir un échantillon de fluide à tester et destinée à être associée à au moins un réactif convenablement couplé à une moitié de paire d'affinité, apte à reconnaître un ligand recherché, et à migrer ;
- une deuxième zone de réaction qui comprend une membrane activée qui est associée à un réactif coloré selon l'une quelconque des revendications 1 à 3, apte à se lier au complexe formé dans la première zone de réaction, et
- une zone de révélation associée à l'autre moitié de paire d'affinité et dans laquelle le complexe éventuellement formé dans la première zone et coloré dans la deuxième zone est fixé.

**24°)** Dispositif selon la revendication 23, caractérisé en ce qu'entre la première et la deuxième zone de réaction, une zone de contrôle du bon fonctionnement du test est prévue et comprend une phase solide convenable contenant un réactif de référence.

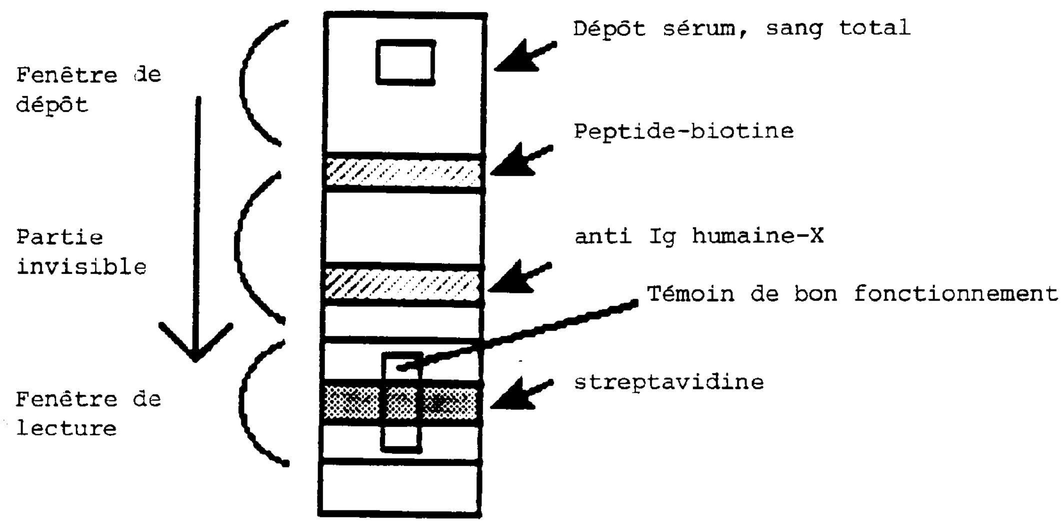
Fenêtre de dépôt
Partie invisible
Fenêtre de lecture
Dépôt sérum, sang total
Peptide-biotine
anti Ig humaine-X
Témoin de bon fonctionnement
streptavidine

FIGURE 1

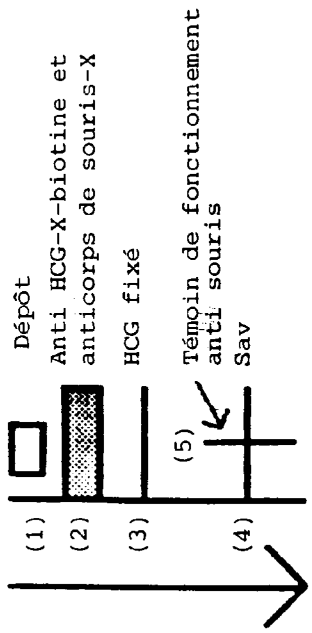


2b

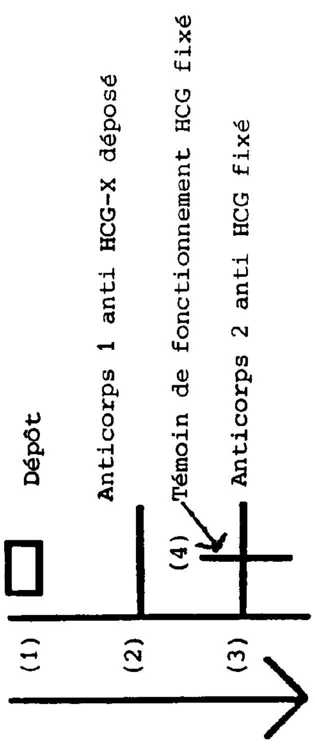


2a

FIGURE 2

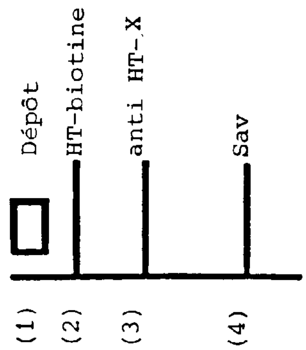


3b

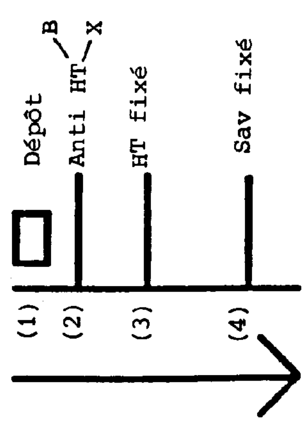


3a

FIGURE 3

Office européen des brevets

# RAPPORT DE RECHERCHE EUROPEENNE

Numero de la demande
EP 92 40 3052

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 064 833 (THE PUBLIC HEALTH LABORATORY SERVICE BOARD)<br>* page 3, ligne 16 - ligne 32; revendications 2,3 * | 1-9 | G01N33/533<br>G01N33/58<br>G01N33/543 |
| Y | GB-A-2 081 257 (ABBOTT LABORATORIES)<br>* le document en entier * | 1-9 | |
| Y | EP-A-0 015 519 (FUJI PHOTO FILM CO., LTD)<br>* page 5, ligne 23 - page 6A, ligne 10 *<br>* page 6B, ligne 10 - page 7, ligne 20 * | 1-9 | |
| A | EP-A-0 201 633 (ABBOTT LABORATORIES)<br>* page 16, ligne 35 - page 17, ligne 7; figures 10,11,12 * | 1-6 | |
| A | WO-A-9 007 950 (JOHN W. EATON)<br>* revendications 3,4 * | 1-9 | |
| A | US-A-4 743 551 (S. SUBRAMANIAN) | | |

DOMAINES TECHNIQUES RECHERCHES (Int. Cl.5)

G01N

Le présent rapport a été établi pour toutes les revendications

| Lieu de la recherche | Date d'achèvement de la recherche | Examinateur |
|---|---|---|
| LA HAYE | 01 FEVRIER 1993 | CARTAGENA ABELLA P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)